Europäisches Patentamt

European Patent Office

Office européen des brevets

(19)

(11) **EP 1 082 597 B1**

(12) **EUROPÄISCHE PATENTSCHRIFT**

(45) Veröffentlichungstag und Bekanntmachung des Hinweises auf die Patenterteilung:
**11.08.2004 Patentblatt 2004/33**

(51) Int Cl.[7]: **G01N 3/08**, B01J 2/22
// (A61J3/02, B02C4:10)

(21) Anmeldenummer: **99919015.0**

(22) Anmeldetag: **14.05.1999**

(86) Internationale Anmeldenummer:
**PCT/CH1999/000204**

(87) Internationale Veröffentlichungsnummer:
**WO 1999/061885 (02.12.1999 Gazette 1999/48)**

(54) **VERFAHREN UND VORRICHTUNG ZUR BESTIMMUNG DES VERDICHTUNGSFAKTORS VON PULVERN**

METHOD AND DEVICE FOR DETERMINING THE COMPRESSION FACTOR OF POWDERS

PROCEDE ET DISPOSITIF POUR LA DETERMINATION DU FACTEUR DE COMPRESSION DE POUDRES

(84) Benannte Vertragsstaaten:
**AT BE CH DE DK ES FR GB IT LI NL SE**

(30) Priorität: **23.05.1998 CH 112598**

(43) Veröffentlichungstag der Anmeldung:
**14.03.2001 Patentblatt 2001/11**

(73) Patentinhaber: **Gerteis, Paul**
**8645 Jona (CH)**

(72) Erfinder: **Gerteis, Paul**
**8645 Jona (CH)**

(74) Vertreter: **Herrmann, Peter Johannes, Dr. et al**
**Patentanwalt**
**Meiersmattstrasse 30**
**6043 Adligenswil/Lu (CH)**

(56) Entgegenhaltungen:
EP-A- 0 447 156    WO-A-91/16982
WO-A-92/13633    US-A- 3 750 467
US-A- 5 140 861

- **PATENT ABSTRACTS OF JAPAN vol. 015, no. 262 (P-1222), 3. Juli 1991 (1991-07-03) -& JP 03 085425 A (BABCOCK HITACHI KK), 10. April 1991 (1991-04-10)**
- **PATENT ABSTRACTS OF JAPAN vol. 015, no. 320 (P-1238), 15. August 1991 (1991-08-15) -& JP 03 115833 A (BABCOCK HITACHI KK), 16. Mai 1991 (1991-05-16)**

**Beschreibung**

Die Erfindung betrifft ein Verfahren zur Bestimmung des Verdichtungsfaktors von Pulvern zur Verarbeitung mit Walzenpressen mit Walzendurchmessern von 100 bis 400 mm.

Walzenpressen dienen zum Verpressen von Pulvern zu Schülpenbändern, welche anschliessend zu Granulat zerkleinert werden (EP-A-0 525 135). Dieser Prozess wird als Trockengranulationsverfahren bezeichnet. Das Verpressen des Pulvers findet zwischen 2 gegenläufigen Walzen statt, wobei je nach Hersteller beide Walzen fix gelagert sind, d.h. einen fixierten Walzenspalt aufweisen, oder eine der beiden Walzen ist beweglich ausgeführt, wird mit hydraulischer Kraft beaufschlagt und verfügt über einen variablen Walzenspalt.

Alle Walzenpressen verfügen über mindestens 2 Presswalzen und werden je nach Hersteller mit verschiedenen Walzenabmessungen angeboten. Die Beschickung der Walzen mit Pulver erfolgt entweder durch die Schwerkraft oder durch Unterstützung einer Stopfschnecke.

Einen besonders wirtschaftlichen Stellenwert erfährt das Trockengranulationsverfahren in der pharmazeutischen Industrie für die Herstellung von Granulat anstelle der bekannten Feuchtgranulationsverfahren.

In der ersten Entwicklungsphase eines neuen Medikamentes musste bis anhin auf die Entwicklung mit dem Ziel der Trockengranulation als kostengünstigste Produktionsmethode verzichtet werden, weil die benötigten Pulvermengen für einen Versuch nicht vorhanden oder zu teuer sind. Ebenfalls fehlt an den meisten Walzenpressen eine entsprechende Instrumentierung, um verwertbare Daten für die gezielte Entwicklung zu erhalten.

Eine geeignete Vorrichtung ist nach der EP-A-0 525 135 bekannt. Diese Einrichtung liefert mit Pulvermengen ab 100 ml bereits reproduzierbare Resultate.

In der ersten Entwicklungsphase stehen nur wesentlich kleinere Mengen, (z.B. 10 ml) für Versuche zur Verfügung. In diesem Stadium muss das spätere Herstellungsverfahren bereits festgelegt werden.

Aus mit bekannten Methoden hergestellten Granulat können Tabletten oder Kapseln hergestellt werden. Die wichtigsten technologischen Eigenschaften, wie der typische Einzugswinkel und daraus resultierend der mögliche verdichtungsfaktor des Pulvers sowie die Produktsmenge pro Zeiteinheit lassen sich mit dem Tablettieren nicht feststellen.

Aufgabe der Erfindung ist es, ein Verfahren bereit zu stellen, um ein Pulver auf seine Eignung zur Herstellung von Granulat nach dem Trockengranulatverfahren mit Walzenpressen im technischen Massstab zu untersuchen.

Eine weitere Aufgabe ist es, eine Vorrichtung zur Durchführung des Verfahrens zur Verfügung zu stellen.

Die Aufgabe wird erfindungsgemäss nach dem Kennzeichen von Anspruch 1 gelöst.

Das erfindungsgemässe Verfahren hat den Vorteil, dass mit einer einzigen Presswalze in Kombination mit einer Matrize für kleinste Pulvermengen eine Vorbestimmung zum Trockengranulierung auf Walzenpressen aller Art erstmals möglich ist. Es erlaubt, die wichtigen Daten für eine zuverlässige Voraussage zum Trockengranulationsverfahren mit Walzenpressen mit minimalstem Materialaufwand zu erzielen und die benötigte Pulvermenge für einen Versuch so gering als möglich zu halten.

Durch die Simulation der zweiten Walze wurde die benötigte Menge grundsätzlich halbiert. Durch den Wegfall der Stopfschnecke konnte eine schmale Presswalze gewählt werden. Um die Messung für alle gängigen Walzenpressen mit unterschiedlichen Konzeptionen durchführen zu können, verfügt die erfindungsgemässe Einrichtung über verschiedene Messysteme.

Als besonders vorteilhaft hat sich erwiesen ein in einer Nut eigefülltes Pulver in einem darin vordefiniertem Spalt zu verdichten.

Es ist zweckmässig, das in der Nut eingefüllte Pulver mit einer vordefinierten Kraft zu verdichten.

Es ist besonders vorteilhaft, die Nut in einer verschiebbaren Matrize auszuführen. Durch diese Anordnung kann auf eine zweite Presswalze verzichtet werden.

Es ist zweckmässig, ein in der Nut vorgesehener Kolben über ein Führungsteil und eine Führungsstange zu bewegen und an einem Lineal und einem Zeiger eine Wegstrecke abzulesen, welcher einen Messwert darstellt.

Es wird bei fest vordefiniertem Walzenspalt die resultierende Presskraft gemessen.

Es wird bei fest vordefinierter Presskraft der resultierende Walzenspalt gemessen.

Bei der Vorrichtung ist der rotierenden Presswalze eine Nut in einer verschiebbaren Matrize und ein Kolben über einen Führungsteil und eine Führungsstange, einem Lineal und einem Zeigerzugeordnet. Das hat den Vorteil, dass nur eine einzige Presswalze benötigt wird, wodurch die Vorrichtung sehr einfach ist. Diese Vorrichtung ist zur Bestimmung des Verdichtungsfaktor von Pulvern geeignet, unabhängig von der Provenienz einer Walzenpresse.

Der typische Einzugswinkel ist bei Walzenpressen aufgrund der paarweisen Anordnung der Presswalzen jeweils an der linken wie auch der rechten Walze aktiv, wird aber für die Beurteilung nur einmal benötigt.

Die Masseinheit für die höchste, auf das Pulver momentan ausgeübte Presskraft ist: kN/cm lineare Walzenbreite. Diese Kraft wird an einer theoretischen Linie quer zur Walze an der Stelle appliziert wo sich die beiden Walzen am nächsten sind. Um möglichst wenig Pulver zu benötigen, wendet dieses Verfahren Walzen mit einer Breite von 1 cm an. Es können auch breitere Walzen zu Vergleichsmessungen wie z.B. für die Scale up Validierung eingesetzt werden.

Um das Verhalten bei verschiedenen Walzendurchmessern testen zu können, kann das Gegenlager an verschiedenen Positionen montiert werden, damit Presswalzendurchmesser von 100 bis 400 mm der unterschiedlichen Hersteller simuliert werden können.

Die Erfindung soll anhand einer Zeichnung näher beschrieben werden. Es zeigen:

Fig. 1    die erfindunggemässe Vorrichtung im Querschnitt zur Messung bei definiertem Walzenspalt und resultierender Presskraft

Fig. 2    eine Variante der erfindungsgemässen Vorrichtung zur Messung bei definierter Presskraft und resultierendem Walzenspalt.

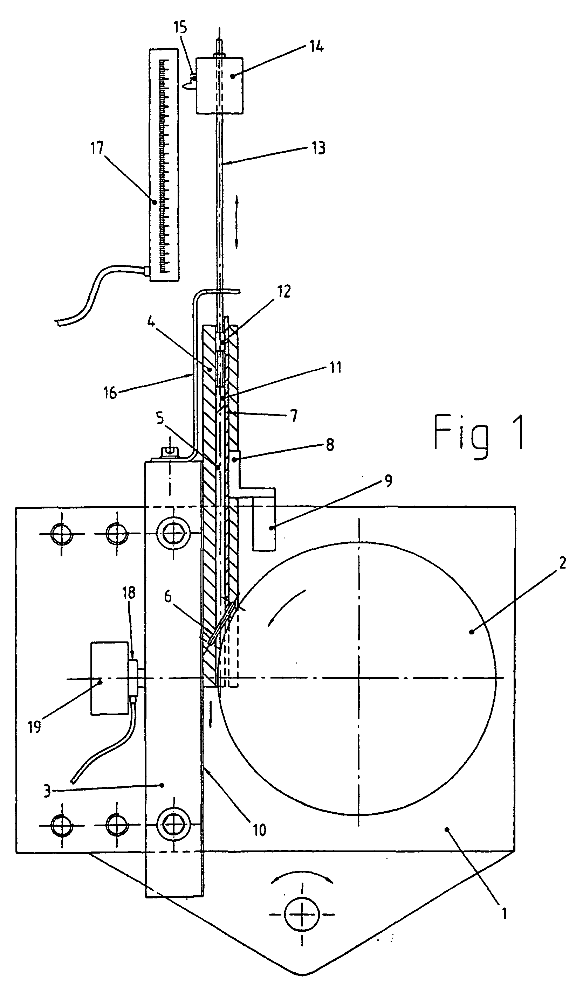

In Fig. 1 ist ein Lagerblock mit dem Bezugszeichen 1 bezeichnet. In dem Lagerblock 1 befindet sich die nicht sichtbare, mit einem Motor angetriebene Antriebswelle auf welcher eine austauschbare Presswalze 2 montiert ist. Am Lagerblock 1 ist ebenfalls ein in verschiedenen Positionen montierbare Gegenlager 3 als feststehender Träger für eine in der Dicke variable Gleitfläche 10 montiert. Eine Matrize 4 ist mit einer Nut 5 versehen, welche breiter als die Presswalze 2 ist. Die Tiefe der Nut 5 ist grösser als ein Winkeltangens von 20 Grad an der Presswalze gemessen aus der horizontalen Mittelachse. Ein tangential zur Walzenoberfläche verlaufender Querschieber 6 dichtet die Matrize 4 nach unten ab und verhindert das unerwünschte Ausfliessen des Pulvers vor der Messung. Die Nut 5 wird mit einem Längsschieber 7 abgedeckt. Ein Anschlag 8 wird am Klemmstück 9 in seiner Höhenlage positioniert. Ein Kolben 11 dichtet die Nut 5 nach oben ab. Ein Führungsteil 12 verhindert ein Verkanten des Kolbens 11 in der Nut 5. Ein Führungsteil 12 und ein Kolben 11 sind auf der Führungsstange 13 montiert. Ein tariertes Gewicht 14 mit einem fest installierten Zeiger 15 ist am oberen Ende der Führungsstange 13 montiert. Ein Halter 16 stabilisert die Führungsstange 13 in ihrer vertikalen Lage. Eine Skala 17 erlaubt es die jeweilige Lage des Kolbens vor und nach dem Prozess abzulesen und für die Berechnung auszuwerten. Die Wegmessung kann auch elektronisch oder optisch erfolgen. Eine Kraftmesszelle 18 ist zwischen den Rücken des Gegenlagers 3 und einer Halterung 19 montiert.

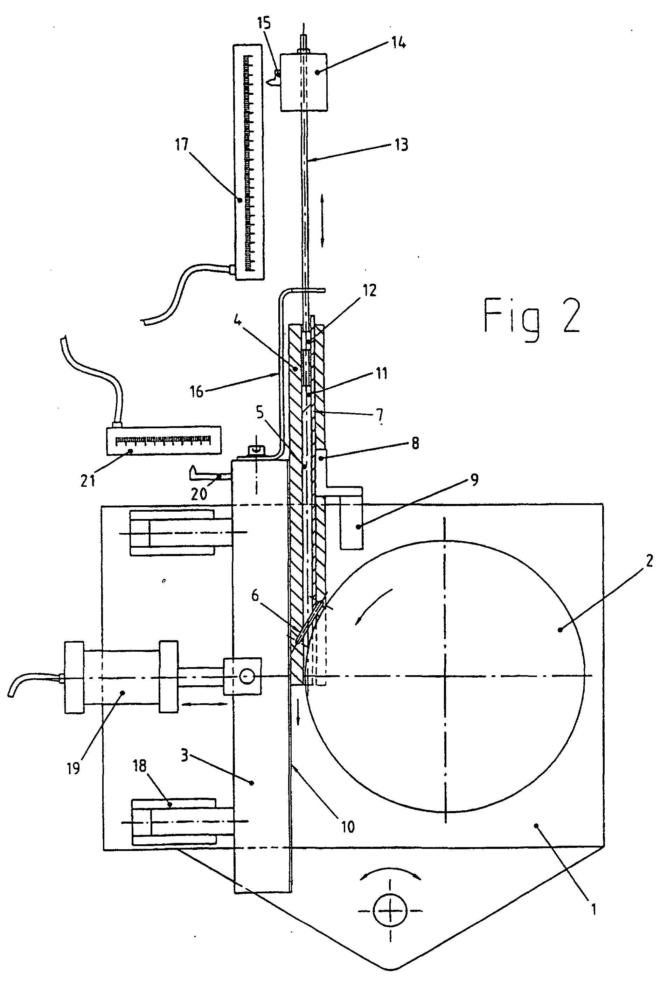

In Fig. 2 ist das Verfahren von Walzenpressen, die mit einer fest definierten Presskraft und einem variablen Walzenspalt versehen sind simuliert. Bei dieser Variante der Vorrichtung wird zusätzlich die Verschiebung des Gegenlagers 3 relativ zur Presswalze über einen Zeiger 20 auf einer Skala 21 angezeigt und abgelesen. Die Wegmessung kann auch elektronisch oder optisch erfolgen.

Die Vorrichtung kann in jeder Ausführung in einem Winkel zwischen 0 und 90 Grad aus der horizontalen Achse angeordnet sein damit alle gebräuchlichen Walzentypen simuliert werden können.

Für die Messung sind 2 Verfahren gebräuchlich.

Messverfahren 1:

Es kann sowohl lose geschüttetes Pulver, d.h. Grundlage ist das Schüttvolumen eines Pulvers oder einer Pulvermischung auf das Kompaktierverhalten getestet werden oder nach

Messverfahren 2

Die Matrize mit dem lose geschüttetem, Pulver oder Pulvermischung auf einem kalibrierten Stampfvolumimeter mit 1250 Hüben nach anerkanntem Verfahren zur Bestimmung des Stampfvolumens vorverdichtet werden.

Im Betrieb wird für die Befüllung die Matrize 4 horizontal auf eine saubere geeignete Unterlage gelegt. Die nach oben eröffnete Nut 5 wird mit Pulver gefüllt.

Nach dem Einfüllen des Pulvers in die Nut 5 der Matrize 4 werden der Querschieber 6 und der Längsschieber 7 in ihre Führungen geschoben und der Kolben 11 eingeführt. Durch das Einführen der Querschieber 6 und des Längsschiebers 7 und des Kolbens 11 wird der Pulverraum geschlossen. Die Matrize 4 wird angehoben und auf die Presswalze 2 geschoben bis der Querschieber 6 die Walzenoberfläche berührt und die Matrize an ihrer hinteren geschlossenen Seite am Gegenlager 3 anliegt. Der Querschieber 6 wird jetzt entfernt und die Matrize 4 soweit nach unten auf die Presswalze 2 geschoben bis das Pulver an der Walzenoberfläche anliegt. Gleichzeitig wird der Anschlag 8 des Längsschieber 7 auf der Auflage 9 anliegen. Zu diesem Zeitpunkt wird die Lage des Zeigers 15 gegen die Skala 17 tariert und somit der Nullpunkt gesetzt.

Definition des Messvorgangs:

Ist der zurückgelegte Weg am Umfang der vorwärts rotierenden Presswalze 2 gleich dem Weg der, sich nach unten bewegenden Matrize 4 und des Kolbens 11, bedeutet dies, dass sich die gesamte eingefüllte Pulvermenge um den Faktor verdichtet hat, der sich aus der folgenden Gleichung ergibt:

Beispiel:

Walzenweg am Umfang 100 mm
Matrizenweg       100 mm + Kolben relativ zur Matrize = 0 mm
Fülltiefe Nut     =     5 mm
Walzenspalt    =     1 mm

Errechnet sich aus der Formel:

$$\frac{5}{1} : \frac{100}{100 \quad 0} \text{ ein Verdichtungsfaktor} = 5$$

Der Einzugswinkel kann jetzt aus einer Tabelle abgelesen werden.

Ein Differenzwert zwischen dem Weg des Kolbens 11 und dem am Umfang der Presswalze 2 (welcher gleich dem Weg der Matrize 4 ist, entsteht, wenn das Pulver aufgrund seines veränderten Einzugsverhaltens einen niedrigeren Einzugswinkel erlaubt und somit einen Rückstosswert am Kolben 11 verursacht. Dieser Rückstosswert wird über den Kolben 11, die Führungsstange 13, den Zeiger 15 am Lineal 17 angezeigt und abgelesen. Der Rückstosswert wird für die Berechnung des Verdichtungsfaktors zum Weg der Matrize hinzuaddiert.

Es ergibt sich folgende Formel

z.B.

Walzenweg am Umfang 100 mm

Matrizenweg      100 mm + Kolben relativ zur Matrize = 50 mm

Fülltiefe Nut     =     5 mm

Walzenspalt     =     1 mm

Errechnet sich aus der Formel

$$\frac{5}{1} : \frac{100}{100 \quad 50} \text{ein Verdichtungsfaktor = 3,333}$$

Der Einzugswinkel kann jetzt aus einer Tabelle abgelesen werden.

In diesem Fall wurde nicht alles Pulver in der Nut 5 eingezogen und verdichtet, sondern nur dasjenige Pulver, das sich im Bereich eines geringeren Einzugswinkels befand.

Während des Messvorgangs wird gleichzeitig die, auf die Kraftmesszelle 18 ausgeübte Kraft sowie das aufgebrachte Drehmoment an der Antriebswelle der Presswalze 2 aufgezeichnet. Die ausgeübte Presskraft ergibt direkt einen Wert der auf Produktionsmaschinen übertragbar ist, d.h.KN/cm lin.

Das aufgebrachte Drehmoment ergibt einen Wert der auf Produktionsmaschinen direkt übertragbar ist, wenn die effektive Breite der Presswalzen in cm mit dem gemessenen Wert multipliziert wird. Sinnvoller ist jedoch auch diesen Wert in Nm/cm Walzenbreite zu definieren.

Das aus der Matrize entfernte gepresste Pulverband kann jetzt weiter zu Granulat verarbeitet und zu einer oder mehreren Tabletten gepresst oder in Kapseln abgefüllt werden. Alternativ kann das Pulverband in einem separaten, genormten Tablettenbohrapparat an verschiedenen Stellen nach einem festgelegten Verfahren angebohrt werden. Die erhaltenen Werte geben über eine Vergleichstabelle Aufschluss über die Zugfestigkeit des Pulverbandes, was wiederum vergleichbar ist mit einer entsprechenden Tablettenhärte.

Um das Verhalten bei definierter Presskraft und resultierendem Walzenspalt testen zu können, ist das Gegenlager 3 an einer Linearführung 18 an montiert.

Es befindet sich in dem Lagerblock 1 die nicht sichtbare, mit einem Motor angetriebene Antriebswelle, auf welcher die austauschbare Presswalze 2 montiert ist. Am Lagerblock 1 ist an einem in verschiedenen Positionen montierbarer Hydraulikzylinder 19 als beweglicher Träger für das Gegenlager 3 und die Gleitfläche 10 montiert. Die ebenfalls in verschiedenen Positionen montierbaren Linearführungen 18 stabilisieren das Gegenlager 3 relativ zu Presswalzenachse.

Die Matrize 4 ist mit einer Nut 5 versehen, welche breiter als die Presswalze 2 ist. Die Tiefe der Nut 5 ist grösser als ein Winkeltangens von 20 Grad an der Presswalze gemessen aus der horizontalen Mittelachse. Ein tangential zur Walzenoberfläche verlaufender Querschieber 6 dichtet die Matrize 4 ab und verhindert das unerwünschte Ausfliessen des Pulvers vor der Messung. Die Nut 5 wird mit einem Längsschieber 7 abgedeckt. Ein Anschlag 8 wird am Klemmstück 9 in seiner Höhenlage positioniert. Ein Kolben 11 dichtet die Nut 5 nach oben ab. Ein Führungsteil 12 verhindert ein Verkanten des Kolbens 11 in der Nut 5. Der Führungsteil 12 und der Kolben 11 sind auf der Führungsstange 13 montiert. Ein tariertes Gewicht 14 mit einem fest installierten Zeiger 15 ist am oberen Ende der Führungsstange 13 montiert. Ein Halter 16 stablilisert die Führungsstange 13 in ihrer vertikalen Lage. Eine Skala 17 erlaubt es die jeweilige Lage des Kolbens vor und nach dem Prozess abzulesen und für die Berechnung auszuwerten. Die Wegmessung kann auch elektronisch oder optisch erfolgen.

Der Hydraulikzylinder 19 wird mit einem definiertem Druck beaufschlagt, aus der Summe, Druck x Fläche des Hydraulikzylinders errechnet sich die applizierte Kraft, die auf das Gegenlager wirkt. Um das Pulver kompaktieren zu können, muss beim Einziehen die, vom Hydraulikzylinder applizierte Kraft überwunden werden und das Gegenlager 3 in den Linearführungen verschoben werden. Der Wert um den sich das Gegenlager 3 verschiebt wird über das Lineal 20 auf die Skala 21 übertragen und als Dicke des gepressent Pulvers abgelesen.

Für die Messung sind 2 Verfahren gebräuchlich.

Messverfahren 1:

es kann sowohl lose geschüttetes Pulver, d.h. Grundlage ist das Schüttvolumen eines Pulvers oder einer Pulvermischung auf das Kompaktierverhalten getestet werden oder nach Messverfahren 2, die Matrize mit dem lose geschüttetem, Pulver oder Pulvermischung auf einem kalibrierten Stampfvolumimeter mit 1250 Hüben nach anerkanntem Verfahren zur Bestimmung des Stampfvolumens vorverdichtet werden.

Für die Befüllung wird die Matrize 4 horizontal auf eine saubere geeignete Unterlage gelegt. Die nach oben geöffnete Nut 5 wird mit Pulver gefüllt. Nach dem Einfüllen des Pulvers in die Nut 5 der Matrize 4 werden der Querschieber 6 und der Längsschieber 7 in ihre Führungen geschoben und der Kolben 11 eingeführt. Durch das Einführen der Querschieber 6 und des Längsschiebers 7 und des Kolbens 11 wird der Pulverraum geschlossen. Die Matrize 4 wird angehoben und auf die Presswalze 2 geschoben bis der Querschieber 6 die Walzenoberfläche berührt und die Matrize 4 an ihrer hinteren geschlossenen Seite am Gegenlager 3 anliegt. Der Querschieber 6 wird jetzt entfernt und die Matrize 4 soweit nach unten auf die Presswalze 2 geschoben bis das Pulver an der Walzenoberfläche anliegt. Gleichzeitig wird der Anschlag 8 des Längsschieber 7 auf der Auflage 9 aufliegen. Zu diesem Zeitpunkt wird die Lage des Zeigers 15 gegen die Skala 17 tariert und somit der Nullpunkt gesetzt. Der Hydraulikzylinder wird mit einem definierten Druck über eine nicht gezeigte Pumpe vorgespannt.

Die Anzeige am Lineal 21 wird gegen den Zeiger 20 auf 0 gesetzt und tariert. Die Wegmessung kann auch elektronisch oder optisch erfolgen.

Gemäss Fig. 2 ist der zurückgelegte Weg am Umfang der vorwärts rotierenden Presswalze 2 gleich dem Weg der, sich bewegenden Matrize 4 und des Kolbens 11. Das bedeutet, dass sich die gesamte eingefüllte Pulvermenge um den Faktor verdichtet hat, der sich aus der folgenden Gleichung ergibt:

Beispiel:

Walzenweg am Umfang 100 mm
Matrizenweg 100 mm + Kolben relativ zur Matrize = 0 mm
Fülltiefe Nut = 5 mm
Anzeige Walzenspalt am Lineal (21) 1 mm

Errechnet sich aus der Formel

$$\frac{5}{1} : \frac{100}{100 \quad 0} \text{ ein Verdichtungsfaktor} = 5$$

Der Einzugswinkel kann jetzt aus einer Tabelle abgelesen werden.
Die applizierte Presskraft KN/ cm lin. war als feste Grösse vordefiniert und kann direkt auf Produktionsmaschinen übertragen werden.

Ein Differenzwert zwischen dem Weg des Kolbens 11 und dem am Umfang der Presswalze 2, welcher gleich dem Weg der Matrize 4 ist, entsteht, wenn das Pulver aufgrund seines veränderten Einzugsverhaltens einen niedrigeren Einzugswinkel erlaubt und somit einen Rückstosswert am Kolben 11 verursacht. Dieser Rückstosswert wird über den Kolben 11, die Führungsstange 13, den Zeiger 15 am Lineal 17 angezeigt und abgelesen. Der Rückstosswert wird für die Berechnung des Verdichtungsfaktors zum Weg der Matrize hinzuaddiert.

Es ergibt sich folgende Formel
z.B.
Walzenweg am Umfang 100 mm
Matrizenweg 100 mm + Kolben relativ zur
Matrize = 50 mm
Fülltiefe Nut = 5 mm
Walzenspalt = 1 mm

Errechnet sich aus der Formel

$$\frac{5}{1} : \frac{100}{100 \quad 50} \text{ ein Verdichtungsfaktor} = 3,333$$

Der Einzugswinkel kann jetzt aus einer Tabelle abgelesen werden.
In diesem Fall wurde nicht alles Pulver in der Nut (5) eingezogen und verdichtet, sondern nur dasjenige Pulver, das sich im Bereich eines geringeren Einzugswinkels befand.
Die applizierte Presskraft KN/ cm lin. war als feste Grösse vordefiniert, der zurückgelegte Weg des Gegenlagers 3 kann an der Skale 21 als resultierende Spaltbreite abgelesen werden und kann direkt auf Produktionsmaschinen übertragen werden.

Während des Messvorgangs wird gleichzeitig das aufgebrachte Drehmoment an der Antriebswelle der Presswalze 2 aufgezeichnet.

Das aufgebrachte Drehmoment ergibt einen Wert der auf Produktionsmaschinen direkt übertragbar ist, wenn die effektive Breite der Presswalzen in cm mit dem gemessenen Wert mulipliziert wird. Sinnvoller ist jedoch auch diesen Wert in Nm/cm Walzenbreite zu definieren.

Das aus der Matrize entfernte gepresste Pulverband kann jetzt weiter zu Granulat verarbeitet und zu einer oder mehreren Tabletten gepresst oder in Kapseln abgefüllt werden Alternativ kann das Pulverband in einem separaten, genormten Tablettenbohrapparat an verschiedenen Stellen nach einem festgelegten Verfahren angebohrt werden. Die erhaltenen Werte geben über eine Vergleichstabelle Aufschluss über die Zugfestigkeit des Pulverbandes, was wiederum vergleichbar ist mit einer entsprechenden Tablettenhärte.

**Patentansprüche**

1. Verfahren zur Bestimmung des Verdichtungsfaktors von Pulvern zur Verarbeitung mit Walzenpressen mit Walzendurchmessern von 100 bis 400 mm, **dadurch gekennzeichnet, dass** es die folgenden Verfahrensschritte aufweist:

   a) Pulver wird in die Nut einer Matrize eingefüllt;
   b) ein Kolben wird in diese Nut eingeführt;
   c) die Matrize wird auf eine rotierende Presswalze geschoben, bis sie die Walzenoberfläche berührt;
   d) das der Presswalze gegenüberliegende Ende der Matrize wird geöffnet und die Matrize wird soweit nach unten auf die Presswalze geschoben, bis das Pulver an der Walzenoberfläche anliegt;
   e) eine Kraft wird währen der Messung auf den Kolben ausgeübt, um das Pulver zu verdichten;
   f) zur Messung des Verdichtungsfaktors des Pulvers wird die Lage des Kolbens relativ zur Matrize herangezogen.

**2.** Verfahren nach Anspruch 1, **dadurch gekennzeichnet, dass** die rotierende Presswalze (2) das in die Nut (5) eingefüllte Pulver in dem vordefinierten Spalt verdichtet.

**3.** Verfahren nach Anspruch 1, **dadurch gekennzeichnet, dass** die rotierende Presswalze (2) das in die Nut (5) eingefüllte Pulver mit einer vordefinierten Kraft verdichtet.

**4.** Verfahren nach den Ansprüchen 1 bis 3, **dadurch gekennzeichnet, dass** die Nut (5) in der verschiebbaren Matrize (4) ausgeführt ist

**5.** Verfahren nach Anspruch 1, **dadurch gekennzeichnet, dass** der in der Nut (5) vorgesehene Kolben (11) über ein Führungsteil (12) und eine Führungsstange (13) bewegt wird und an einem Lineal (17) und einem Zeiger (15) eine Wegstrecke angibt

**6.** Verfahren nach Anspruch 1, **dadurch gekennzeichnet, dass** ein mit einem Gegenlager verbundener Hydraulikzylinder (19) über zwei Linearführungen (18) bewegt wird und an einem Lineal (21) und einem Zeiger (20) eine Wegstrecke angibt.

**7.** Verfahren nach Anspruch 1, **dadurch gekennzeichnet, dass** das Messverfahren bei fest vordefiniertem Walzenspalt die resultierende Presskraft misst.

**8.** Verfahren nach Anspruch 2, **dadurch gekennzeichnet, dass** das Messverfahren bei fest vordefinierter Presskraft die resultierende Spaltbreite misst.

**9.** Vorrichtung zur Durchführung des Verfahrens nach den Ansprüchen 1 bis 7, **dadurch gekennzeichnet, dass** sie eine relativ zu einer rotierenden Presswalze (2) verschiebbare Matrize (4), in der eine Nut (5) ausgeführt ist, enthält, dass ein in dieser Nut verschiebbarer Kolben (11) vorgesehen ist, und dass dieser Kolben über ein Führungsteil (12) und eine Führungsstange (13) mit einem Lineal (17) und einem Zeiger (15) verbunden ist.

**10.** Vorrichtung nach Anspruch 9, **dadurch gekennzeichnet, dass** das Gegenlager (3) an 2 Linearführungen (18) befestigt ist und mit einem Lineal (21) und einem Zeiger (20) verbunden ist

**11.** Vorrichtung nach Anspruch 9, **dadurch gekennzeichnet, dass** die Tiefe der Nut (5) grösser ist als ein Winkeltangens von 20 Grad an der Presswalze (2) gemessen aus der horizontalen Mittelachse.

**Claims**

**1.** Method for determining the compression factor of powders for processing by means of roller presses having roller diameters of 100 to 400 mm, **characterized in** comprising the following steps of:

a) filling a groove of a die with powder;
b) introducing a piston into the groove;
c) pushing the die on a rotating press roller until it touches the roller surface;
d) opening an end of the die located opposite to the press roller and pushing the die downward on the press roller until the powder rests on the roller surface;
e) during the measurement exerting a force on the piston for compressing the powder;
f) and using a position of the piston in relation to the roller for measuring a compression factor.

**2.** Method according to Claim 1, **characterized in that** the rotating press roller (2) compresses a powder introduced in a groove (5) in a predefined nip.

**3.** Method according to Claim 1, **characterized in that** the rotating press roller (2) compresses a powder introduced in a groove (5) with a predefined force.

**4.** Method according to Claims 1 to 3, **characterized in that** the groove (5) is formed in a displaceable die (4).

**5.** Method according to Claim 1, **characterized in that** a piston (11) provided in a groove (5) is moved via a guide part (12) and a guide rod (13) and indicates a travel on a rule (17) and a pointer (15).

**6.** Method according to Claim 1, **characterized in that** a hydraulic cylinder (19) connected to a abutment is moved via two linear guides (18) and indicates a travel on a rule (21) and a pointer (20).

**7.** Method according to Claim 1, **characterized in that** the measuring method measures the resulting press force in the case of a permanently predefined roller nip.

**8.** Method according to Claim 2, **characterized in that** the measuring method measures the resulting nip width in the case of a permanently predefined press force.

**9.** Device for carrying out the method according to Claims 1-7, **characterized in that** the rotating press roller (2) [lacuna] a groove (5) in a displaceable die (4) and a piston (11) via a guide part (12) and a guide rod (13) [lacuna] are connected to a rule (17) and a pointer (15).

**10.** Device according to Claim 9, **characterized in that** abutment (3) is fastened to 2 linear guides (18) and is connected to a rule (21) and a pointer (20).

**11.** Device according to Claim 9, **characterized in that** the depth of the groove (5) is greater than a 20 degree angle tangent to the press roller (2), as measured from the horizontal center axis.

**Revendications**

**1.** Procédé pour déterminer le facteur de compression de poudres en vue de leur traitement avec des presses à rouleaux d'un diamètre de rouleau de 100 à 400 mm, **caractérisé en ce qu'**il comprend les étapes suivantes :

> a) la poudre est versée dans la rainure d'une matrice ;
> b) un piston est introduit dans cette rainure ;
> c) la matrice est amenée sur un rouleau compresseur en rotation, jusqu'à ce qu'elle touche la surface du rouleau ;
> d) l'extrémité de la matrice opposée au rouleau compresseur s'ouvre et la matrice est poussée vers le bas sur le rouleau compresseur jusqu'à ce que la poudre se trouve au niveau de la surface du rouleau ;
> e) une force est exercée sur le piston pendant la mesure afin de comprimer la poudre ;
> f) pour mesurer le facteur de compression de la poudre, on utilise la position du piston par rapport à la matrice.

**2.** Procédé selon la revendication 1, **caractérisé en ce que** le rouleau compresseur en rotation (2) comprime dans l'interstice prédéfini la poudre versée dans la rainure (5).

**3.** Procédé selon la revendication 1, **caractérisé en ce que** le rouleau compresseur en rotation (2) comprime avec une force prédéfinie la poudre versée dans la rainure (5).

**4.** Procédé selon les revendications 1 à 3, **caractérisé en ce que** la rainure (5) est réalisée dans la matrice mobile (4).

**5.** Procédé selon la revendication 1, **caractérisé en ce que** le piston (11) prévu dans la rainure (5) est déplacé par l'intermédiaire d'un élément de guidage (12) et d'une tige de guidage (13) et indique au niveau d'une règle (17) et d'une aiguille (15) une distance.

**6.** Procédé selon la revendication 1, **caractérisé en ce qu'**un cylindre hydraulique (19) relié à un appui est déplacé par l'intermédiaire de deux guides linéaires (18) et indique au niveau d'une règle (21) et d'une aiguille (20) une distance.

**7.** Procédé selon la revendication 1, **caractérisé en ce que** le procédé de mesure mesure la force de compression résultante dans le cas d'un écartement de cylindres bien défini.

**8.** Procédé selon la revendication 2, **caractérisé en ce que** le procédé de mesure mesure la largeur d'écartement résultante dans le cas d'une force de compression bien définie.

**9.** Dispositif pour mettre en oeuvre le procédé selon les revendications 1 à 7, **caractérisé en ce qu'**il comprend une matrice (4) qui est mobile par rapport à un rouleau compresseur en rotation (2) et dans laquelle est réalisée une rainure (5), **en ce qu'**il est prévu un piston (11) mobile dans cette rainure, et **en ce que** ce piston est relié par l'intermédiaire d'un élément de guidage (12) et d'une tige de guidage (13) à une règle (17) et à une aiguille (15).

**10.** Dispositif selon la revendication 9, **caractérisé en ce que** l'appui (3) est fixé à 2 guides linéaires (18) et est relié à une règle (21) et à une aiguille (20).

**11.** Dispositif selon la revendication 9, **caractérisé en ce que** la profondeur de la rainure (5) est supérieure à une tangente angulaire de 20 degrés sur le rouleau compresseur (2), mesurée à partir de l'axe médian horizontal.

Fig 1

Fig 2